# EUROPÄISCHE PATENTSCHRIFT

(11) **EP 2 640 713 B1**
(45) Veröffentlichungstag und Bekanntmachung des Hinweises auf die Patenterteilung: **20.08.2014**
(21) Anmeldenummer: 11779102.0
(22) Anmeldetag: 28.10.2011
(51) Int. Cl.: C07D 307/62

(54) **ASCORBINSÄUREDERIVATE ALS OXIDATIONSFARBSTOFFKOMPONENTEN**
ASCOBIC ACID DERIVATIVES AS COMPONENTS FOR OXIDATION DYES
DÉRIVÉS D'ACID ASCORBIQUE EN TANT QUE COMPOSANTS POUR COLORANTS D'OXYDATION

(30) Priorität: 19.11.2010 EP 10014780
(43) Veröffentlichungstag der Anmeldung: 25.09.2013
(73) Patentinhaber: Merck Patent GmbH, 64293 Darmstadt (DE)
(72) Erfinder: BUEHLE, Philipp, 64673 Zwingenberg (DE); RUDOLPH, Thomas, 64291 Darmstadt (DE)
(86) Internationale Anmeldenummer: PCT/EP2011/005457
(87) Internationale Veröffentlichungsnummer: WO 2012/065685

(56) Entgegenhaltungen:
- WO-A1-2009/097953
- WO-A2-2006/108505

## Beschreibung

Gegenstand der Erfindung ist die Verwendung spezieller Ascorbinsäurederivate zum Färben einer Matrix, wie zum Beispiel Haut, Haare, Nägel oder Textilien bzw. deren Verwendung als Oxidationsfarbstoffkomponenten, spezielle Ascorbinsäurederivate und deren Herstellung. Des Weiteren betrifft die Erfindung ein Verfahren zum Färben einer Matrix mit diesen speziellen Ascorbinsäurederivaten. Ein weiterer Gegenstand der Erfindung ist eine Zubereitung, enthaltend zumindest ein spezielles Ascorbinsäurederivat, sowie ein Verfahren zur Herstellung einer solchen Zubereitung.

Derzeit ist zum Färben von Matrices, wie zum Beispiel Haut, Haare, Nägel oder Textilien, eine Vielzahl von Farbstoffen bekannt. Dabei können sich z.B. Direktfarbstoffe an die Matrix assoziieren und/oder kovalente chemische Bindungen zu der Matrix ausbilden. Bei anderen Färbeprozessen kann eine lösliche Vorstufe des Farbstoffes während des Färbeprozesses zum Farbstoff auf der Matrix umgesetzt werden. Des Weiteren können bei z.B. der Dispersionsfärbung schwer lösliche oder unlösliche Farbstoffe bei Behandlung der Matrix mit einer solchen Dispersion in die Matrix hinein diffundieren und ggf. eine kovalente Bindung zu der Matrix ausbilden. Das Färben der Matrix kann somit in unterschiedlicher Art und Weise stattfinden und zu einem unterschiedlichen Ergebnis hinsichtlich des Anbindungscharakters und auch des Farbergebnisses führen.

Bei sogenannten Oxidationsfarbstoffen, unter anderem beschrieben in "Kosmetik und Hygiene" von Wilfried Umbach, Wiley-VCH, 2004, wird eine Kupplungskomponente (Nuancierer, Kuppler) mit einer Entwicklungskomponente (Oxidationsbase, Entwickler) unter Verwendung von Oxidantien, wie z.B. Wasserstoffperoxid, auf bzw. teilweise in der Matrix zu Farbstoff umgesetzt. Dabei herrschen üblicherweise zumindest während des Färbeprozesses alkalische und oxidative Bedingungen. Das oxidative Milieu ist notwendig, damit die Entwicklungskomponente oxidiert werden kann und in einer Kupplungsreaktion mit der Kupplungskomponente zumindest zu einer Vorstufe eines Farbstoffes reagieren kann. Das alkalische Milieu dient zum einen der Entfaltung der oxidativen Wirkung von z.B. Wasserstoffperoxid und zum anderen der Öffnung der Matrixstruktur, so dass die Kupplungskomponente und/oder die Entwicklungskomponente und/oder daraus entstandene Farbstoffe oder deren Vorstufen tiefer in die Matrix eindringen können. Je tiefer die Komponenten und/oder die Farbstoffe bzw. deren Vorstufen in die Matrix eindringen desto dauerhafter ist die Färbung. Bevorzugt enthalten beide Komponenten unsubstituierte oder substituierte Hydroxy- oder Amingruppen, bevorzugt in ortho-, und paraStellung bei der Entwicklungskomponente und in meta-Stellung bei der Kupplungskomponente. Bei der Bildung eines derartigen Farbstoffes aus der Kupplungskomponente und der Entwicklungskomponente kann zudem eine Anbindung des Farbstoffes an die Matrix stattfinden.

Bei Verwendung von, insbesondere synthetischen, Farbstoffen kann, insbesondere im humanen Anwendungsbereich, zudem eine geringe Verträglichkeit vorliegen. Außerdem wird üblicherweise gerade bei Oxidationsfarbstoffen ein die Matrix stark belastendes alkalisches Milieu verwendet, um die Matrix für die nachfolgende Färbung vorzubehandeln und/oder um während der Färbung den Färbeprozess zu unterstützen. Ebenso ist üblicherweise auch das oxidative Milieu dementsprechend stark ausgebildet.

Es besteht demzufolge weiterhin ein Bedarf an, unter anderem verträglichen und insbesondere hautverträglichen, Farbstoffen oder Oxidationsfarbstoffkomponenten, die sich durch ein schonender durchführbares Färben von Matrices auszeichnen.

Demzufolge beschäftigt sich die vorliegende Erfindung mit dem Problem, verbesserte oder zumindest alternative Oxidationsfarbstoffkomponenten zum Färben von Matrices bereitzustellen, die sich insbesondere durch einen haut-, haarverträglichen sowie schonenden Färbeprozess und eine langanhaltende Färbung der Matrices auszeichnen.

Erfindungsgemäß wird dieses Problem durch die Gegenstände der unabhängigen Ansprüche gelöst. Vorteilhafte Ausführungsformen sind Gegenstand der abhängigen Ansprüche.

Überraschenderweise wurde nun festgestellt, dass sich Ascorbinsäurederivate der Formel I, d.h. in 6- und/oder 5-Position substituierte Ascorbinsäurederivate, in hervorragender Weise als Oxidationsfarbstoffkomponenten zum Färben von Matrices eignen. Dies gilt ebenso für die analogen Dehydroascorbinsäurederivate der Formel I. Dabei kann die Ascorbinsäure bzw. die Dehydroascorbinsäure in dem jeweiligen Ascorbinsäurederivat bzw. Dehydroascorbinsäurederivat in L-Form oder D-Form oder in einer Mischung beider isomerer Formen vorliegen. Vorzugsweise sind die Verbindungen der Formel I in L-Form.

Dabei versteht man unter einer Oxidationsfarbstoffkomponente eine Entwicklungskomponente oder eine Kupplungskomponente eines Oxidationsfarbstoffes. Die Entwicklungskomponente, üblicherweise auch als Entwickler oder Oxidationsbase bezeichnet, wird zusammen mit einer Kupplungskomponente, üblicherweise auch als Nuancierer oder Kuppler bezeichnet, in einem oxidativen Milieu und vorzugsweise alkalischen Milieu zu einem Oxidationsfarbstoff umgesetzt. Dabei wird durch das oxidative Milieu in einem ersten Schritt zumindest die Entwicklungskomponente oxidiert, wobei in einem darauffolgenden Schritt die oxidierte Form der Entwicklungskomponente mit der Kupplungskomponente zu zumindest einer Vorstufe des eigentlichen Oxidationsfarbstoffes reagiert.

Fortfolgend steht jeweils unabhängig voneinander:
Alk für eine geradkettige oder verzweigte oder cyclische C₁- bis C₂₀-Alkylgruppe,
Arl für eine unsubstituierte, einfach- oder mehrfachsubstituierte C₆- bis C₂₀-Arylgruppe, wobei als Substituenten OH, Alk, OAlk und NAlk₂ umfasst sind.

Dabei umfasst eine:
C₁-C₄-Alkylgruppe Methyl, Ethyl, Propyl-, iso-Propyl, Butyl, x-Methylpropyl (x=1;2) und tert.-Butyl;
C₁-C₈-Alkylgruppe die Substituenten der C₁-C₄-Alkylgruppe und Pentyl, Hexyl, Heptyl, Oktyl, x-Methylbutyl (x=1;2;3), x-Methylpentyl (x=1;2;3;4), x-Methylhexyl (x=1;2;3;4;5), x-Ethylpentyl (x=1;2;3), x-Ethylhexyl (x=1;2;3;4), Cyclopentyl, Cyclohexyl, x-Methylcyclopentyl (x=2;3), x-Methylcyclohexyl (x=2;3;4), x-Dimethylcyclopentyl (x=2,3;2,4;3,4), x-Dimethylcyclohexyl (x=2,3;2,4;2,5;3,4;3,5), x-Ethylcyclopentyl (x=2;3) und x-Ethylcyclohexyl (x=2;3;4);
C₁-C₂₀-Alkylgruppe die Substituenten der C₁-C₈-Alkylgruppe und Nonanyl, Decanyl, Undecanyl, Dodecanyl, Tridecanyl, Tetradecanyl, Pentadecanyl, Hexadecanyl, Heptadecanyl, Oktadecanyl, Nonadecanyl und Eicosanyl;
C₆-C₁₂-Arylgruppe Benzyl, Phenyl, Naphthyl und Biphenylyl, C₆-C₁₄-Arylgruppe Substituenten der C₆-C₁₂-Arylgruppe und Anthryl, Phenanthryl und Phenalenyl;
C₆-C₂₀-Arylgruppe die Substituenten der C₆-C₁₄-Arylgruppe und Pyrenyl, Chrysenyl, Naphthacenyl und Picenyl;
einfach- oder mehrfach substituierte C₆-C₁₂-Arylgruppe x-Methylphenyl (x=o,m,p), x-Ethylphenyl (x=o,m,p), x-Propylphenyl (x=o,m,p), x-Isopropylphenyl (x=o,m,p), x-tert.-Butylphenyl (x=o,m,p), x-Methoxyphenyl (x=o,m,p), x-Ethoxyphenyl (x=o,m,p), (x=o,m,p), x,y-Dimethylphenyl (x=2,3;2,4;2,5;2,6;3,4;3,5), x,y-Dihydroxyphenyl (x=2,3;2,4;2,5;2,6;3,4;3,5), x,y-Dimethoxyphenyl (x=2,3;2,4;2,5;2,6;3,4;3,5), 3,4,5-Trimethoxyphenyl oder 2,4,5-Trimethylphenyl,
einfach- oder mehrfach substituierte C₆-C₁₄-Arylgruppe die Substituenten der C₆-C₁₂-Arylgruppe,
einfach- oder mehrfach substituierte C₆-C₂₀-Arylgruppe die Substituenten der C₅-C₁₄-Arylgruppe,
wobei als weitere Substituenten an der jeweiligen Aryl-Gruppe OH, Alk, OAlk oder NAlk₂ auftreten können.

Ein Gegenstand der Erfindung ist somit die Verwendung von Verbindungen der Formel I 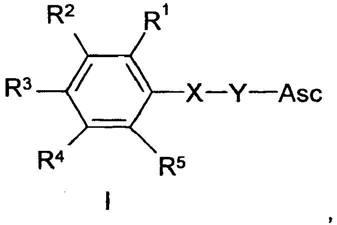
wobei X steht für eine Einfachbindung oder -(CH₂)ₙ-[O-(CH₂)ₘ]ₚ- mit 0≤n≤20, 0≤m≤20, 0≤p≤20 und 0≤n+m·p≤20,
wobei Y steht für eine Einfachbindung oder für -(C=O)-,
wobei Asc steht für einen Ascorbinsäurerest der Formel IIa oder IIb 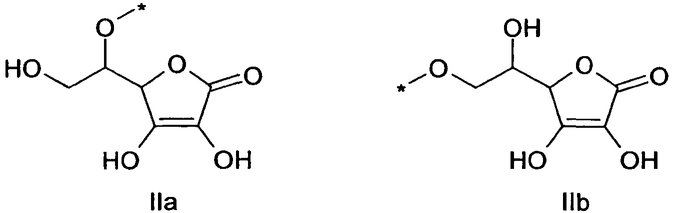
oder einen Dehydroascorbinsäurerest der Formeln IIc oder IId 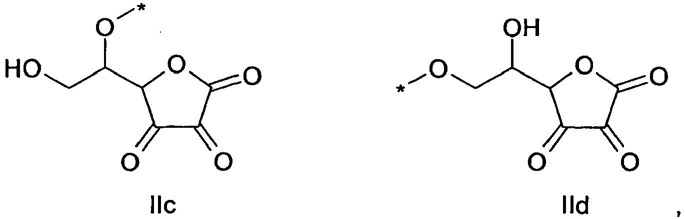
wobei zumindest ein R¹ bis R⁵ steht für NH₂, NHAlk, NAlk₂, NHArl oder NArl₂,
wobei die verbleibenden Substituenten R¹ bis R⁵ jeweils unabhängig voneinander stehen für H, OH, NH₂, Alk, OAlk, NHAlk, NAlk₂, NHArl oder NArl₂,
wobei Alk jeweils unabhängig voneinander steht für eine geradkettige oder verzweigte oder cyclische C₁- bis C₂₀-Alkylgruppe, wobei Arl jeweils unabhängig voneinander steht für eine unsubstituierte, einfach- oder mehrfachsubstituierte C₆- bis C₂₀-Arylgruppe, die mit OH, Alk, OAlk oder NAlk₂ substituiert sein kann,
und/oder deren Salze, Tautomere, Stereoisomere, einschließlich deren Mischungen in allen Verhältnissen, als Oxidationsfarbstoffkomponente zum Färben einer Matrix.

Spezielle Ascorbinsäurederivate sind auf Seite 22 der WO 2008/017346 beschrieben, beispielsweise die in 5- und/oder 6-Position der Ascorbinsäure veresterten Verbindungen mit den Teilstrukturen IVa, IVb und IVe. Die Ascorbinsäurederivate 4-Di-n-hexylaminobenzoesäure-6-O-ascorbat (synonym verwendet auch als 4-Di-hexylaminobenzoesäure-6-O-ascorbat), 4-Di-n-pentylaminobenzoesäure-6-O-ascorbat (synonym verwendet auch als 4-Di-pentylaminobenzoesäure-6-O-ascorbat), 4-Di-(2-Ethylhexyl)aminobenzoesäure-6-O-ascorbat oder 4-Di-n-octylaminobenzoesäure-2-(3,4-dihydroxy-5-oxo-2,5-dihydro-furan-2-yl)-2-hydroxy-ethylester oder 4-Di-laurylaminobenzoesäure-2-(3,4-dihydroxy-5-oxo-2,5-dihydro-furan-2-yl)-2-hydroxy-ethylester sind in WO 2009/097953 beschrieben. In beiden Offenlegungsschriften wird jedoch eine Verwendung dieser speziellen Verbindungen der Formel I als UV-Filter beschrieben, die an eine Matrix binden können.

Dabei stellt die Dehydroascorbinsäure der Formel IV eine oxidierte Form der Ascorbinsäure der Formel III dar, die im menschlichen Organismus wieder zu Ascorbinsäure reduziert werden kann.

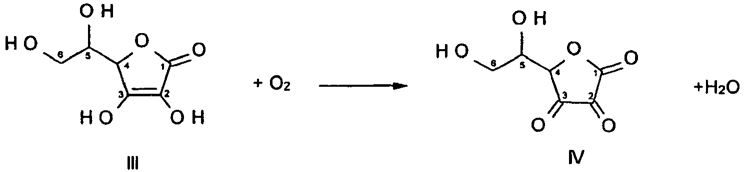

Die Nummerierung der Positionen der Ascorbinsäure sowie der Dehydroascorbinsäure gilt bei den folgenden Bezeichnungen wie in obigem Reaktionsschema dargestellt.

Dabei versteht man unter Matrix eine polymere Verbindung, die als funktionelle Gruppe zumindest eine freie NH-, NH₂-, SH- oder OH-Gruppe aufweist. Unter anderem aufgrund der funktionellen Gruppen der Matrix ist das Farbstoffderivat in der Lage, sich an die Matrix zu assoziieren und gegebenenfalls mit der Matrix kovalente Bindung auszubilden. Bevorzugte Matrices sind hierbei proteinhaltige Matrices. Besonders bevorzugt sind Haut, Haare und/oder Nägel und ganz besonders bevorzugt sind Haare. Des Weiteren sind ebenfalls geeignet synthetische polymere Verbindungen, die zumindest eine der oben aufgeführten funktionellen Gruppen aufweist. Demzufolge lassen sich die Verbindungen der Formel I auch als Oxidationsfarbstoffkomponenten zum Färben von Textilien, einschließlich Kunststofffasern, oder ganz allgemein von Kunststoffen verwenden. Hinsichtlich der Textilfärbung verwendbar sind insbesondere Fasern enthaltend Wolle, Baumwolle oder Seide.

Bevorzugt steht Asc für einen Ascorbinsäurerest nach Formel IIa oder IIb und besonders bevorzugt für einen Ascorbinsäurerest nach Formel IIb. Demzufolge sind die 6-O-Ascorbinsäurederivate besonderes bevorzugt. Diese können auftreten in der D- und/oder L-Form, wobei die L-Form ganz besonders bevorzugt ist.

Eine bevorzugte Ausführungsform ist daher die Verwendung von Verbindungen der Formel I 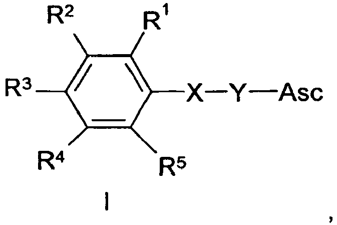
wobei X steht für eine Einfachbindung oder -(CH₂)ₙ-[O-(CH₂)ₘ]ₚ- mit 0≤n≤20, 0≤m≤20, 0≤p≤20 und 0≤n+m·p≤20,
wobei Y steht für eine Einfachbindung oder für -(C=O)-,
wobei Asc steht für einen Ascorbinsäurerest der Formel IIa-1 oder IIb-1 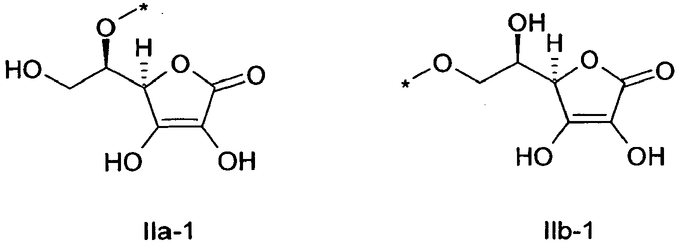
oder einen Dehydroascorbinsäurerest der Formeln IIc-1 oder IId-1 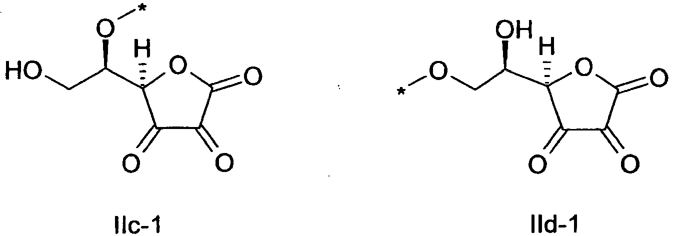
wobei zumindest ein R¹ bis R⁵ steht für NH₂, NHAlk, NAlk₂, NHArl oder NArl₂,
wobei die verbleibenden Substituenten R¹ bis R⁵ jeweils unabhängig voneinander stehen für H, OH, NH₂, Alk, OAlk, NHAlk, NAlk₂, NHArl oder NArl₂,
wobei Alk jeweils unabhängig voneinander steht für eine geradkettige oder verzweigte oder cyclische C₁- bis C₂₀-Alkylgruppe, wobei Arl jeweils unabhängig voneinander steht für eine unsubstituierte, einfach- oder mehrfachsubstituierte C₆- bis C₂₀-Arylgruppe, die mit OH, Alk, OAlk oder NAlk₂ substituiert sein kann,
und/oder deren Salze, Tautomere, Stereoisomere, einschließlich deren Mischungen in allen Verhältnissen, als Oxidationsfarbstoffkomponente zum Färben einer Matrix.

Besonders bevorzugt steht Asc für einen Ascorbinsäurerest nach Formel IIa-1 oder IIb-1 und besonders bevorzugt für einen Ascorbinsäurerest nach Formel IIb-1.

Besonders bevorzugt steht Y für -(C=O)-. Demzufolge sind Verbindungen nach Formel I bevorzugt Ascorbinsäureester.

Bevorzugt ist die Variable m = 0. Bevorzugt ist die Variable p = 0. Demzufolge ist es bevorzugt, wenn X für -(CH₂)ₙ- mit 1≤n≤20 oder für eine Einfachbindung steht. Es ist weiterhin bevorzugt, wenn die Variable n 1, 2, 3 oder 4 bedeutet, d.h. es ist bevorzugt, dass X für -(CH₂)ₙ- mit 1≤n≤4 oder eine Einfachbindung steht und ganz besonders bevorzugt für -(CH₂)- (d.h. n=1) oder für eine Einfachbindung steht.

Bevorzugt stehen die Substituenten R¹ bis R⁵ jeweils unabhängig voneinander für H, Alk, OH, NH₂, NHAlk, NAlk₂, NHArl oder NArl₂, besonders bevorzugt für H, NH₂, NHAlk, NAlk₂, NHArl oder NArl₂ und ganz besonders bevorzugt für H oder NH₂.

Bevorzugt stehen die Substituenten R² und R⁵, sofern sie zu den verbleibenden Substituenten gehören, für H oder Alk, besonders bevorzugt für H. Besonders bevorzugt steht der Substituent R⁴ für H oder Alk, besonders bevorzugt für H.

Bevorzugt steht Alk jeweils unabhängig voneinander für eine geradkettige oder verzweigte oder zumindest teilweise cyclische C₁-C₈-Alkylgruppe, besonders bevorzugt für eine geradkettige oder verzweigte C₁-C₄-Alkylgruppe oder für Hexyl, Cyclohecyl oder Ethylhexyl.

Bevorzugt steht Arl für eine unsubstituierte, einfach- oder mehrfachsubstituierte C₆- bis C₂₀-Arylgruppe, wobei als Substituenten OH, Alk, OAlk und NAlk₂ umfasst sind, besonders bevorzugt für eine unsubstituierte, einfach- oder mehrfachsubstituierte C₆- bis C₁₄-Arylgruppe, wobei als Substituenten OH, Alk und OAlk umfasst sind, ganz besonders bevorzugt für eine unsubstituierte, einfach- oder mehrfachsubstituierte C₆- bis C₁₀-Arylgruppe, wobei als Substituenten OH, Alk und OAlk umfasst sind.

Da Verbindungen der Formel I als Oxidationsfarbstoffkomponenten verwendet werden, können sie demzufolge als Kupplungskomponenten oder als Entwicklungskomponenten eingesetzt werden. Bevorzugt ist dabei die Verwendung als Entwicklungskomponenten.

Weil Verbindungen der Formel I zudem einen Ascorbinsäure- bzw. Dehydroascorbinsäurerest aufweisen, der als Linker fungieren kann, können Verbindungen der Formel I mittels des Linkers an die jeweilige Matrix assoziert werden und/oder mittels des Linkers eine kovalente Bindung zur jeweiligen Matrix ausbilden. Im Vergleich zu herkömmlichen Oxidationsfarbstoffkomponenten, insbesondere im Vergleich zu herkömmlichen Entwicklungskomponenten, weisen somit Verbindungen der Formel I eine gesteuerte Anbindung an die Matrix auf, weshalb sich eine Färbung von Matrices mit diesen erfindungsgemäßen Oxidationsfarbstoffkomponenten schonender durchführen lässt. Da nun bei Verbindungen der Formel I zusätzlich eine Anbindung über den Linker Ascorbinsäure- bzw. Dehydroascorbinsäurerest an die jeweilige Matrix vorliegt, kann ein geringer alkalisches Milieu verwendet werden, da eine hohe Tiefenwirkung durch ein stark alkalisches Milieu nun nicht mehr derartig ausgeprägt benötigt wird, um eine dauerhafte Färbung zu erzielen. Die Dauerhaftigkeit der Färbung kann nun zumindest teilweise durch die Anbindung der Verbindungen der Formel I über ihren Linker an die Matrix bewerkstelligt werden. Das Öffnen der Matrix durch ein stark alkalisches Milieu und das Eindringen von Farbstoffen in die geöffnete Matrix ist somit nicht mehr zwingend notwendig. So kann gegebenenfalls gänzlich auf ein alkalisches Milieu verzichtet werden. Wird dennoch zumindest ein schwach alkalisches Milieu verwendet, dann kann mit Verbindungend der Formel I eine durchdringendere Färbung des Haares erreicht werden.

Durch Einsatz von nur einem Ascorbinsäure- bzw. Dehydroascorbinsäurerest als Linker sind Verbindungen der Formel I zudem amphiphil, weshalb die Penetration der Verbindungen der Formel I in die Matrix leichter stattfindet und eine Anbindung zumindest der Verbindungen der Formel I an die Matrix verstärkt ist. Auch deshalb kann ein geringer alkalisches Milieu beim Färben und/oder während einer Vorbehandlung der Matrix verwendet werden und das Färben schonender durchgeführt werden.

Auch wird aufgrund der hohen Amphiphilie und des großen Wasserbindungsvermögens der Verbindungen der Formel I die Feuchtigkeit einer damit behandelten Matrix deutlich verbessert. Dies führt zu verbesserten Matrixstruktureigenschaften und zu einer erhöhten Elastizität der Matrix.

Bezogen auf die besonders bevorzugte Matrix Haar kann gesagt werden, dass Verbindungen der Formel I geeignet sind, um durch die Haarkutikula in den Haarkortex vorzudringen, wodurch eine vollständige und anhaltende Haarfärbung über das gesamte Haar erzielbar wird. Des Weiteren verbessern die Verbindungen der Formel I die Haarfeuchtigkeit durch hervorragende Hydratisierung des Haarkeratins. Mit der Haarfeuchtigkeit einhergehend verbessern sich somit die Haarstruktureigenschaften, insbesondere die Haarelastizität.

Verbindungen der Formel I können durch ein zugegebenes Oxidationsmittel, wie z.B. Wasserstoffperoxid, oxidiert werden. Verbindungen der Formel I können auch durch den eigenen Ascorbinsäurerest der Formel IIa, IIb, IIa-1 oder IIb-1 oder durch einen Dehydroascorbinsäurerest der Formel IIc, IId, IIc-1 oder IId-1 oder durch den Ascorbinsäurerest der Formel IIa, IIb, IIa-1 oder IIb-1 oder durch einen Dehydroascorbinsäurerest mindestens einer weiteren Verbindung der Formel I oxidiert werden. Dabei kann mit anderen Worten ein Molekül einer Verbindung der Formel I eine Autooxidation erfahren oder durch ein weiteres Molekül einer Verbindung der Formel I oxidiert werden. So ist es auch denkbar, dass Verbindungen der Formel I als Kupplungskomponenten eingesetzt werden, die eine andere Entwicklungskomponente oxidieren. Bei der Verwendung von Verbindungen der Formel I kann somit gegebenenfalls auf ein separat zugegebenes Oxidationsmittel verzichtet werden. Wird ein Oxidationsmittel verwendet, kann die Färbung intensiviert werden.

Bevorzugt kann zumindest eine Verbindung der Formel I als Entwicklungskomponente eingesetzt werden. Somit wird zumindest eine Verbindung der Formel I während des Färbevorganges oxidiert und die oxidierte Form der zumindest einen Verbindung der Formel I mit zumindest einer Kupplungskomponente zu einem Oxidationsfarbstoff oder dessen Vorstufe umgesetzt. Vorteilhaft findet dabei eine gezielte Anbindung der Entwicklungskomponente, gegebenenfalls vor der Oxidation, durch den Linker Ascorbinsäurerest der Formel IIa, IIb, IIa-1 oder IIb-1 bzw. Dehydroascorbinsäurerest der Formel IIc, IId, IIc-1 oder IId-1 statt.

Besonders im Fall der Verwendung einer Verbindung der Formel I, wie zuvor beschrieben oder als bevorzugt beschrieben, als Entwicklungskomponente wird die Anbindung des Linkers Asc nach Formel IIa, IIb, IIa-1, IIb-1 IIc, IId, IIc-1 oder IId-1 durch die oxidative Aktivierung zudem noch intensiviert und beschleunigt.

Gleichzeitig sind Verbindungen der Formel I, wie zuvor beschrieben oder als bevorzugt beschrieben, wegen ihrer Ascorbinsäureabstammung sehr gute Antioxidantien. Dies ist von Vorteil, da Färbeprozeduren, insbesondere Haarfärbeprozesse, unter Bedingungen, z.B. alkalischen Bedingungen in Gegenwart von Oxidationsmitteln wie Wasserstoffperoxid und/oder Ammoniumperoxid, ablaufen, die zur Ausbildung reaktiver Sauerstoff- und/oder Stichstoff und/oder Kohlenstoffspecies führen (z.B. ROS oder synonym reactive oxygen species). Diese können anhaftende Gewebe wie Wolle, Haut oder Haar schädigen. Antioxidantien wirken diesem Effekt entgegen.

Ein weiterer Gegenstand der Erfindung ist ein Färbeverfahren einer insbesondere proteinhaltigen Matrix, bei dem in einem Färbeschritt die Matrix mit einer Dispersion und/oder einer Lösung und/oder einer Emulsion behandelt wird, die zumindest eine Verbindung der Formel I, wie zuvor beschrieben als Entwicklungskomponente, aufweist, wobei während des Färbeschrittes zumindest eine Kupplungskomponente mit zumindest einer Verbindung der Formel I oder gegebenenfalls mindestens einer weiteren Entwicklungskomponente zu einem die Matrix färbenden Oxidationsfarbstoff oder dessen Vorstufe reagiert.

Bekannte Kupplungskomponenten und Entwicklungskomponenten werden nachfolgend beschrieben.

Ein weiterer Gegenstand der Erfindung ist ein Färbeverfahren einer insbesondere proteinhaltigen Matrix, bei dem in einem Färbeschritt die Matrix mit einer Dispersion und/oder einer Lösung und/oder einer Emulsion behandelt wird, die zumindest eine Verbindung der Formel I, wie zuvor beschrieben als Kupplungskomponente, aufweist, wobei während des Färbeschrittes zumindest die Verbindung der Formel I mit einer Entwicklungskomponente zu einem die Matrix färbenden Oxidationsfarbstoff oder dessen Vorstufe reagiert. Auch bei diesem Färbeverfahren kann gegebenenfalls mindestens eine weitere bekannte Kupplungskomponente beteiligt sein.

Dabei kann gegebenenfalls in einem Vorbehandlungsschritt die Matrix zur Beeinflussung und insbesondere zur Verbesserung des Färbeverhaltens derselben mittels eines Vorbehandlungsmittels vorbehandelt werden. Ein solches Vorbehandlungsmittel kann basisch, sauer oder neutral sein, eine oxidative Wirkung, zum Beispiel durch Vorhandensein eines Oxidationsmittels wie Wasserstoffperoxid, aufweisen und gegebenenfalls Wasser enthalten. Bevorzugt weist das Vorbehandlungsmittel NH₃, (NH₄)CO₃ und/oder H₂O₂ auf. Üblicherweise wird der Vorbehandlungsschritt vor dem Färbeschritt durchgeführt, es ist aber auch eine gleichzeitige Durchführung von Vorbehandlungsschritt und Färbeschritt bei dementsprechender Formulierung denkbar.

Da die Verbindungen der Formel I aufgrund ihres Linkers eine gute Anbindungs- und Penetrationsfähigkeit an die Matrix besitzen, kann demzufolge das Milieu nicht nur während des Färbeschrittes, sondern auch während des Vorbehandlungsschrittes schwächer oxidativ und/oder alkalisch eingestellt werden, um eine vergleichbare Dauerhaftigkeit der Färbung zu erzielen. Es kann gegebenenfalls sogar auf ein oxidatives und/oder alkalisches Milieu verzichtet werden.

Ein weiterer Gegenstand der Erfindung sind neue Verbindungen der Formel I, wie zuvor beschrieben, umfassend auch die besonders bevorzugten Definitionen der involvierten Variablen Asc, X, Y, R¹ bis R⁵, mit der Maßgabe, wenn X für eine Einfachbindung steht und Y für -(C=O)- steht, zumindest ein Substituent R¹ bis R⁵ für NHArl oder NArl₂ steht.

Verbindungen der Formel I weisen genau einen Ascorbinsäurerest der Formel IIa, IIb, IIa-1 oder IIb-1 oder einen Dehydroascorbinsäurerest der Formel IIc, IId, IIc-1 oderr IId-1 auf und einen Rest einer aromatischen Verbindung der Formel V, wie nachfolgend beschrieben. Durch den Ascorbinsäurerest bzw. Dehydroascorbinsäurerest sind die Verbindungen der Formel I, insbesondere für den Menschen, verträglicher und somit in der Regel hinsichtlich ihrer Verträglichkeit, insbesondere für den Menschen, gegenüber Verbindungen der Formel V, wie nachfolgend beschrieben, verbessert.

Ein weiterer Gegenstand der Erfindung ist ein Verfahren zur Herstellung einer Verbindung der Formel I, wie zuvor beschrieben, bei dem in einem Veresterungsschritt eine COOH-Gruppe (Z = OH) oder in einem Ether-Bildungsschritt eine Abgangsgruppe Z einer aromatischen Verbindung der Formel V (Z = Cl, Br, I, OTosyl, OMesyl), 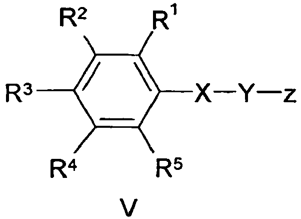
wobei X eine vorhergehend beschriebene Bedeutung hat,
wobei Y eine vorhergehend beschriebene Bedeutung hat,
wobei Z eine Abgangsgruppe ist beispielsweise Cl, Br, I, OH, OTosyl, OMesyl oder Z eine Hydroxygruppe ist,
wobei die verbleibenden Substituenten R¹ bis R⁵ eine vorhergehend beschriebene Bedeutung haben,
mit genau einer Hydroxygruppe auf der fünften oder sechsten Position der Ascorbinsäure der Formel III oder der Dehydroascorbinsäure der Formel IV umgesetzt wird.

Die Ascorbinsäure der Formel III kann in der D- oder L-Form oder in einer Mischung dieser beiden Formen vorliegen. Bevorzugt entspricht die Ascorbinsäure der Formel III der L-Form.
Die Dehydroascorbinsäure der Formel IV kann in der D- oder L-Form oder in einer Mischung dieser beiden Formen vorliegen. Bevorzugt entspricht die Dehydroascorbinsäure der Formel IV der L-Form.

Mesyl bedeutet Methylsulfonyl.
Tosyl bedeutet 4-Toluolsulfonyl.

Somit wird der Linker Ascorbinsäure oder Dehydroascorbinsäure über eine seiner OH-Gruppen der fünften und/oder sechsten Position mit der aromatischen Verbindung der Formel V durch Veresterung oder Veretherung verbunden. Erfindungsgemäß kommen deshalb aromatische Verbindungen der Formel V in Frage, die entweder, falls Y steht für -(C=O) und Z steht für OH, eine Carbonsäuregruppe, oder, falls Y steht für eine Einfachbindung, Z eine Abgangsgruppe aufweisen, sowie deren Salze oder aktivierte Formen, wie zum Beispiel Aktivester, Säurechloride oder dergleichen.

Vor dem Veresterungsschritt kann in einem Aktivierungsschritt die COOH-gruppe der jeweiligen aromatischen Verbindung der Formel V für eine nachfolgende Veresterung aktiviert werden. Zu diesem Zweck kann aus einer aromatischen Verbindung der Formel V ein Aktivester, ein Säurechlorid oder dergleichen hergestellt werden.

Des Weiteren kann vor dem Veresterungsschritt und ggf. vor dem Aktivierungsschritt zumindest eine Hydroxygruppe auf der fünften oder sechsten Position der Ascorbinsäure oder der Dehydroascorbinsäure mittels einer ersten Schutzgruppe geschützt werden. Ebenso kann des Weiteren zumindest eine Hydroxygruppe auf der zweiten oder dritten Position der Ascorbinsäure durch eine zweite Schutzgruppe geschützt werden.

Nach dem Veresterungsschritt kann zumindest eine erste und/oder eine zweite Schutzgruppe abgespalten werden, wobei die erste und die zweite Schutzgruppe unter unterschiedlichen Reaktionsbedingungen abspaltbar ist.

Als Schutzgruppen können die üblicherweise bekannten und oftmals verwendeten Schutzgruppen wie zum Beispiel, Methoxymethyl-, Benzyl-, SilylGruppen oder dergleichen zum Einsatz kommen.

Durch Derivatisierung der Verbindungen der Formel V mit Ascorbinsäure bzw. Dehydroascorbinsäure zeichnen sich die Verbindungen der Formel I zudem durch eine höhere biologische Abbaubarkeit aus und sind somit umweltverträglicher als die Verbindungen der Formel V.

Ein weiterer Gegenstand der Erfindung sind Zubereitungen enthaltend zumindest einen für kosmetische, pharmazeutische, dermatologische Zubereitungen oder Haushaltsprodukte geeigneten Träger und zumindest eine Verbindung der Formel I, wie zuvor beschrieben.

Die Zubereitung kann dabei zusätzlich zu dem zumindest einen für kosmetische, pharmazeutische, dermatologische Zubereitungen oder Haushaltsprodukte geeigneten Träger zumindest ein Vorbehandlungsmittel zur Beeinflussung des Färbeverhaltens der zu färbenden Matrix aufweisen.

Bevorzugt ist die Zubereitung als Mehrkomponentensystem ausgestaltet, wobei zumindest eine Verbindung nach Formel I, wie zuvor beschrieben, als Entwicklungskomponente, zumindest eine Kupplungskomponente, optional ein Vorbehandlungsmittel, optional eine weitere Entwicklungskomponente und/oder zumindest ein Oxidationsmittel auf zumindest zwei Zubereitungskomponenten verteilt sind. Bevorzugt weist eine erste Zubereitungskomponente zumindest eine Verbindung nach Formel I und zumindest eine Kupplungskomponente und zumindest einen für kosmetische, pharmazeutische, dermatologische Zubereitungen oder Haushaltsprodukte geeigneten Träger und gegebenenfalls zumindest ein nicht-oxidatives Vorbehandlungsmittel auf, während eine zweite Kupplungskomponente zumindest ein Oxidationsmittel, insbesondere Wasserstoffperoxid aufweist.

Ein weiterer Gegenstand der Erfindung ist ein Verfahren zur Herstellung einer Zubereitung, wie zuvor beschrieben, wobei zumindest ein Verbindung nach Formel I, wie zuvor beschrieben, mit zumindest einem für kosmetische, pharmazeutische, dermatologische Zubereitungen oder Haushaltsprodukte geeigneten Träger und gegebenenfalls Hilfsstoffen und/oder Füllstoffen gemischt, insbesondere dispergiert und/oder emulgiert und/oder gelöst wird.

Des Weiteren können, um z.B. weitere Farbanpassungen vornehmen zu können, die Verbindungen der Formel I mit vorbekannten Oxidationsfarbstoffkomponenten kombiniert werden.

Geeignete Oxidationsfarbstoffkomponenten vom Entwicklertyp sind p-Phenylendiamin und dessen Derivate. Geeignete p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Weitere geeignete p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Als weitere geeignete Entwicklerkomponenten können Verbindungen eingesetzt werden, die mindestens zwei aromatische Kerne enthalten, die mit Amino- und/oder Hydroxylgruppen substituiert sind. Weitere geeignete Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetra-methylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Weitere geeignete zweikernige Entwicklerkomponenten werden ausgewählt aus N,N-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es möglich sein, als Entwicklerkomponente ein p-Aminophenolderivat oder eines seiner physiologisch verträglichen Salze einzusetzen. Bevorzugte p-Aminophenole sind p-Aminophenol, N-Methylp-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus PyrimidinDerivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Weitere geeignete Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydro-xyethyl)pyrazol. Als Pyrazolopyrimidine sind insbesondere Pyrazolo[1,5-a]pyrimidine geeignet, wobei bevorzugte Pyrazolo[1,5-a]pyrimidine ausgewählt sind aus Pyrazolo[1,5-a]pyrimidin-3,7-diamin, 2,5-Dimethyl-pyra-zolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyra-zolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Weitere geeignete Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,M-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Weitere geeignete Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1 H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,0001 bis 10 Gew.-%, vorzugsweise 0,001 bis 5 Gew.-%, jeweils bezogen auf die anwendungsbereite Zubereitung oder Zubereitungskomponente, verwendet.

Geeignete Oxidationsfarbstoffkomponenten vom Kupplertyp werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Di- beziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Weitere verwendbare Kupplerkomponenten, wie m-Aminophenole bzw. deren Derivate, werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamitio)benzol, 3-Ethylamino-4-methylphenol, 2,4-Dichlor-3-aminophenol und deren physiologisch verträglichen Salzen.

Weitere verwendbare Kupplerkomponeten, wie z.B. 3-Diaminobenzole bzw. deren Derivate, werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methyiphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Weitere verwendbare Kupplerkomponeten, wie z.B. o-Diaminobenzole bzw. deren Derivate, werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Weitere verwendbare Kupplerkomponeten, wie z.B. Di- beziehungsweise Trihydroxybenzole und deren Derivate, werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Weitere verwendbare Kupplerkomponeten, wie z.B. Pyridinderivate, werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimetho-xypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Als Kupplerkomponente geeignete Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Als Kupplerkomponente geeignete Indolderivate werden ausgewählt aus 4-Hydroxyindol, 6-Hydroxyindol und 7-Hydroxyindol und deren physiologisch verträglichen Salzen.

Als Kupplerkomponente geeignete Indolinderivate werden bevorzugt ausgewählt aus 4-Hydroxyindolin, 6-Hydroxyindolin und 7-Hydroxyindolin und deren physiologisch verträglichen Salzen.

Als Kupplerkomponente geeignete Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Geeignete Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethylamino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydro-xyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 10 Gew.-%, vorzugsweise 0,001 bis 5 Gew.-%, jeweils bezogen auf die anwendungsbereite Zubereitung oder Zubereitungskomponente, verwendet.

Bei den Zubereitungen handelt es sich dabei üblicherweise um Zubereitungen zur Anwendung an Textilien oder zur Anwendung an Haut und Haar von Mensch und Tier, besonders bevorzugt zur Anwendung an Haar. Beispielhaft sind kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Weitere bevorzugte Kombinationen von Ausführungsformen sind in den Ansprüchen offenbart.

Gegenstand der Erfindung ist auch ein Verfahren zur Herstellung einer Zubereitung, wie zuvor beschrieben, wobei mindestens eine Verbindung nach Formel I, wie zuvor beschrieben oder als bevorzugt beschrieben, mit einem Träger und gegebenenfalls mit weiteren Aktiv- oder Hilfsstoffen vermischt wird. Geeignete Trägerstoffe sowie Aktiv- oder Hilfsstoffe sind im nachfolgenden Teil ausführlich beschrieben.

In bevorzugten Ausführungsformen wird die mindestens eine Verbindung nach Formel I mit den definierten oder als bevorzugt angegebenen Substituenten oder bevorzugte Einzelverbindungen, insbesondere den Verbindungen 1a, 1b, 1c oder 1d, in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

In den beschriebenen Zubereitungen, die erfindungsgemäß mindestens eine Verbindung nach Formel I enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Die Zubereitungen können neben den Verbindungen nach Formel I sowie den gegebenenfalls anderen Inhaltsstoffen weitere organische UV-Filter, sogenannte hydrophile oder lipophile Sonnenschutzfilter, die im UVA-Bereich und/oder UVB-Bereich und(/oder IR und/oder VIS-Bereich (Absorber) wirksam sind enthalten. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische wie auch anorganische UV Filter sind in den Patentanmeldungen EP-A 0 487 404 sowie WO2009/077356 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung

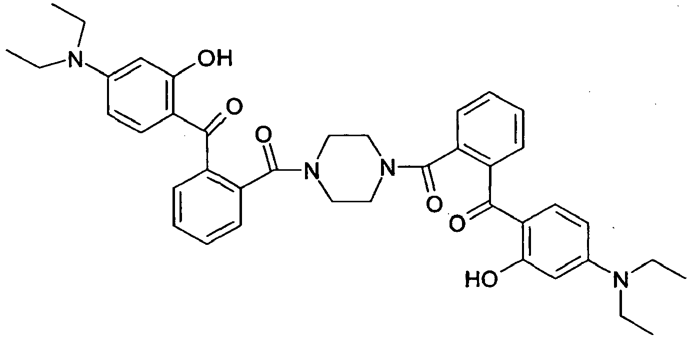

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylene-bis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls anderen organischen UV-Filtern, wie zuvor beschrieben, weitere anorganische UV-Filter, sogenannte partikuläre UV-Filter enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt einzusetzende partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination einzusetzenden behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   ∘ "Zinc Oxide CS-5" der Fa. Toshibi (ZnO nachbehandelt mit polymethylhydrogenosiloxane)
   ∘ Nanogard Zinc Oxide FN der Fa. Nanophase Technologies
   ∘ "SPD-Z1" der Fa Shin-Etsu (ZnO nachbehandelt mit einem Silikongepfropften Acrylpolymer, dispergiert in Cyclodimethylsiloxane
   ∘ "Escalol Z100" der Fa ISP (Aluminiumoxid nachbehandeltes ZnO dispergiert in einer ethylhexyl methoxycinnamate/PVPhexadecene/methicone copolymer Mischung)
   ∘ "Fuji ZNO-SMS-10" der Fa. Fuji Pigment (ZnO nachbehandelt mit Siliciumdioxid und Polymethylsilesquioxan);
   ∘ Unbehandeltes Ceroxide Mikropigment z.B. mit der Bezeichnung "Colloidal Cerium Oxide" der Fa Rhone Poulenc
   ∘ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Daher kann es bevorzugt sein, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Ferner sind erfindungsgemäße Farbstoffe mit allen Wirkstoffen und Hilfstoffen kombinierbar, wie sie in WO2009/09819 systematisch gelistet sind. Insbesondere gehören diese Stoffe zu den darin aufgeführten Verwendungskategorien "Moisturizers and Humectants", "Desquamating agents", "Agents for improving the barrier function", "Depigmenting Agents", "Antioxidants", "Dermo-relaxing or dermo-decontracting agents", "Anti-glycation agents", "Agents for stimulating the synthesis of dermal and/or epidermal macromolecules and/or for preventing their degradation", "Agents for stimulating fibroblast or keratinocyte proliferation and/or keratinocyte differentiation", "Agents for promoting the maturation of the horny envelope", "NO-synthase inhibitors", "Peripheral benzodiazepine receptor (PBR) antagonists", "Agents for increasing the activity of the sebaceous glands", "Agents for stimulating the energy metabolism of cells", "Tensioning agents", "Fat-restructuring agents", "Sliming agents", "Agents for promoting the cutaneous microcirculation", "Calmatives or anti-irritants", "Sebo-regulating or anti-seborrhoic agents", "Astringents", "Cicatrizing agents", "Anti-inflammatory agents", "Antiacne agents".

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B 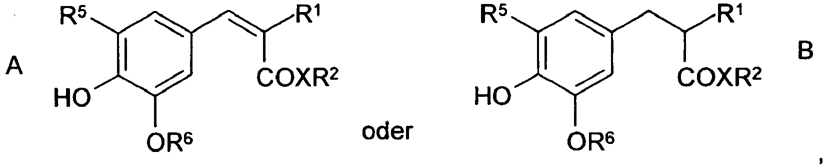 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils ungabhängig voneinander H oder lineares oder verzweigtes Alkyl mit 1 bis 8 C-Atomen,
R⁵H oder lineares oder verzweigtes Alkyl mit 1 bis 8 C-Atomen oder lineares oder verzweigtes Alkoxy mit 1 bis 8 C-Atomen und
R⁶lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxyne^{®} ST Liquid) und/oder 2-(4-Hydroxy-3,5-dimethoxybenzyl)-malonsäure-bis-(2-ethylhexyl)ester (z.B. RonaCare^{®} AP). Ferner ist die Kombination mit 2-(4-Hydroxy-3-methoxybenzyliden)-malonsäure-bis-isopropylester bzw. 2-(4-Hydroxy-3-methoxybenzyl)-malonsäure-bis-isopropylester (hydrogenated diisopropyl vanilidene malonate) bevorzugt. Analoges gilt für entsprechende bis-ethylester.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), β-Mannosylglycerat (Firoin), β- Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine oder die Handelsprodukte Ronacare®soquercetin, Ronacare®Tilirosid oder Ronacare®Cyclopeptide 5 verwendet werden.

Ferner können die erfindungsgemäßen Zubereitungen mindestens einen Selbstbräuner als weiteren Inhaltsstoff enthalten.
Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin oder Rucinol.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellutosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Iso-propanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methylcyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzwegten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die erfindungsgemäße Mischung kann in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasserin-Öl (W/O) oder vom Typ (Ol-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol-(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane, bevorzugt Alkane.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Beispiele:

### Beispiel 1: Synthese von 4-Amino-benzoesäure-(R)-2-((R)-3,4-dihydroxy5-oxo-2,5-dihydro-furan-2-yl)-2-hydroxy-ethylester (Verbindung der Formel 1a).

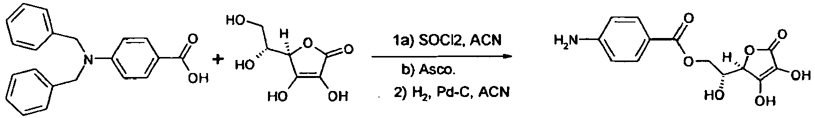

Es werden 5 g 4-Dibenzylamino-benzoesäure (15,6 mmol) in 75 ml Acetonitril gelöst und bei 0°C 2,36 g Thionylchlorid (18,97 mmol, 1,2 Äq.) langsam zugetropft. Nach 30 min Reaktion bei Raumtemperatur werden 4,16 g Ascorbinsäure (23,6 mmol, 1,5 Äq.) portionsweise zugegeben, wobei die Temperatur bei max. 30°C gehalten wird. Nach 60 min Reaktionszeit werden 84 mg Pd-C (10%ig Pd, 0,005 Äq.) zugegeben und Wasserstoff bei 2 bar zugegeben. Nach 180 min Reaktionszeit wird der Katalysator über Aktivkohle abfiltriert und das Lösungsmittel im Vakuum entfernt. Der Rückstand wird in 200 ml Ethylacetat aufgenommen, mit 2 x 100 ml ges. Kochsalzlösung extrahiert, 100 ml Ethylacetat abdestilliert und anschließend mit Heptan bei -5°C das Produkt ausgefällt, mit 50 ml kaltem Heptan gewaschen und getrocknet. Man erhält 3,1 g Produkt (67%) als weißen Feststoff.

Analog wird die Verbindung 3-Amino-benzoesäure-(R)-2-((R)-3,4dihydroxy-5-oxo-2,5-dihydro-furan-2-yl)-2-hydroxy-ethylester **(1 b)** aus 3-Dibenzylamino-benzoesäure hergestellt.

Analog wird die Verbindung 2-Amino-benzoesäure-(R)-2-((R)-3,4dihydroxy-5-oxo-2,5-dihydro-furan-2-yl)-2-hydroxy-ethylester **(1c)** aus 2-Dibenzylamino-benzoesäure hergestellt.

Analog wird die Verbindung 4-Amino-phenylessigsäure-(R)-2-((R)-3,4dihydroxy-5-oxo-2,5-dihydro-furan-2-yl)-2-hydroxy-ethylester **(1d)** aus 4-Dibenzylamino-phenylessigsäure hergestellt.

### Beispiel 2: Herstellung einer Färbecreme

Folgende Färbecremes werden hergestellt:

| Rohstoffe | Gew% |
|---|---|
| Farbpulvermischung | 2,3 |
| Ammonium Carbomer, 1 % | 12,0 |
| Sodium Cetearyl Sulfate | 0,6 |
| Sodium Laureth Sulfate | 3,5 |
| Kalium Oleat, 12,5% | 2,4 |
| Glyceryl Stearate | 1,6 |
| Glycol Distearate | 1,6 |
| Octyldodecanol | 1,6 |
| Cetearyl Alcohol | 9,6 |
| Ceteareth-20 | 2,4 |
| L-Serie | 0,5 |
| Linoleamidopropyl PGdimonium chloride phosphate | 0,1 |
| Na4-EDTA, Pulver, 87% | 0,2 |
| Ascorbinsäure | 0,1 |
| Natrium sulfit, wasserfrei, 96% | 0,2 |
| Ammoniak, 25% | 6,0 |
| Parfum | qs |
| Wasser | ad 100 |

Dabei wird eine Farbpulvermischung, enthaltend 59,2 Gew.-% der Ascorbinsäurederivate nach Beispiel 1; 6,6 Gew.-% 3-Aminophenol; 22,7 Gew.-% Resorcin; 0,8 Gew.-% 2-Amino-4-hydroxyethylaminoanisolsulfat und 10,7 Gew.-% Siliciumdioxid, pyrogen; jeweils bezogen auf das Gesamtgewicht der Farbpulvermischung verwendet.

Die Fettbasis wird jeweils zusammen bei 80°C aufgeschmolzen und mit einem Teil der Wassermenge dispergiert. Anschließend werden die restlichen Rezepturbestandteile unter Rühren der Reihe nach eingearbeitet. Dann wird mit Wasser auf 100 Gew.-% aufgefüllt und die Formulierung kalt gerührt.

Vermischen mit der Entwicklerdispersion (EW) und Applikation:
Jede Färbecreme wird im Gewichtsverhältnis 1:1 mit einer wie folgt zusammengesetzten Entwicklerdispersion ausgemischt.

| Rohstoff | Gew.-% |
|---|---|
| Na-benzoat | 0,04 |
| Dipicolinsäure | 0,10 |
| Dinatriumpyrophosphat | 0,19 |
| 1,2-Propandiol | 0,50 |
| HEDP, 60% | 0,25 |
| Paraffinum Liquidum | 0,30 |
| Steartrimonium Chloride (~80%ig) | 0,20 |
| Cetearyl Alcohol | 3,00 |
| Eumulgin B 2 | 0,70 |
| Wasserstoffperoxid 50% | 12,2 |
| Kaliumhydroxid, 50% | 0,19 |
| Wasser | ad 100 |

Strähnen von natürlich dunklem Haar (natural dark European Alkinco 6634) werden mit 10 Gew.-% Natrium-Laurylethersulfat-Lösung im Ultraschallbad für 15 min behandelt, anschließend 10 min mit lauwarmen gespült. Die Strähnen wurden an der Luft getrocknet und für 24 h bei 25°C und 40% relativer Luftfeuchtigkeit gelagert.

Für die Färbung wurde auf den Strähnen von ca. 0,7 g Gewicht die 4-fache Menge der fertigen Anwendungsmischungen appliziert. Nachdem die Strähnen für 30 min bei 32°C gefärbt werden, werden sie für 5 min mit Wasser gespült und an der Luft getrocknet.

### Färbeergebnis:

Mit den Färbezubereitungen werden abhängig von der verwendeten erfindungsmäßigen Oxidationsfarbstoffkomponente satte Haarfärbungen erzielt.

### Beispiel 3: Versuche zur Kupplungsreaktion der Ascorbinsäurederivate. Die Verbindung der Formel 1 a, 1 b, 1 c, 1 d

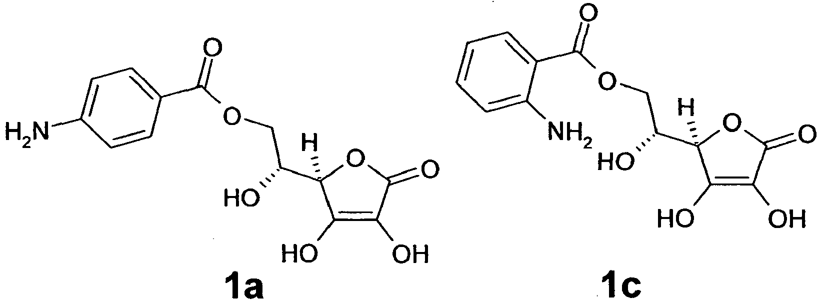

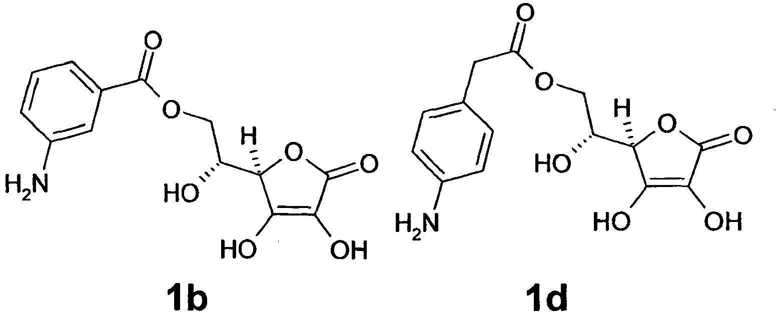

werden in einer Reaktion zur oxidativen Farbstoffbildung umgesetzt.

Von der jeweiligen Verbindung 1a, 1b, 1c und 1d werden jeweils 4%ige wässrige Lösungen hergestellt. Der pH-Wert dieser Lösungen bewegt sich zwischen 6.0 und 6.3, also im schwach sauren Bereich. Zu einem Teil dieser wässrigen Lösungen wird jeweils das gleiche Volumen einer 6%igen H₂O₂-Lösung gegeben und 5 min oxidiert. Anschließend erfolgt die Zugabe einer 4%igen Lösung einer beispielhaften Kupplungskomponente (Resorcin / pH = 4.8) zur wässrigen Lösung als auch zur Lösung enthaltend H₂O₂. Nach 30 min Reaktionszeit wird die Farbreaktion optisch begutachtet. Es kann festgestellt werden, dass die Verbindungen 1a, 1b, 1c und 1d sowohl unter oxidativen, als auch rein wässrigen Bedingungen nach Zugabe der Kupplungskomponente eine deutliche Färbung zeigen. Dabei kann folgende Farbvertiefung und/oder Farbverschiebung beobachtet werden:

| | Start | 5 min nach Zugabe von | | 30 min nach Zugabe von Resorcin zur | |
|---|---|---|---|---|---|
| | | Wasser | H₂O² | wässr. Lsg | Lsg enth. H₂O₂ |
| 1a | farblos | farblos | farblos | rot | orange |
| 1b | rosa | rosa | rosa | dunkelrot | rot |
| 1c | gelb | hellrosa | Hellrosa/gelb | rot | dunkelorange |
| 1d | gelb | gelb | gelb | dunkelrot | rotbraun |

## Patentansprüche

1. Verwendung von Verbindungen der Formel I 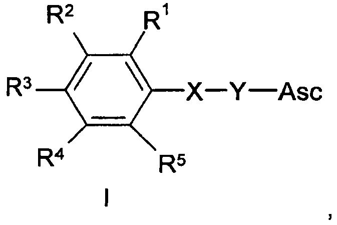
wobei X steht für eine Einfachbindung oder -(CH₂)ₙ-[O-(CH₂)ₘ]ₚ- mit 0≤n≤20, 0≤m≤20, 0≤p≤20 und 0≤n+m·p≤20,
wobei Y steht für eine Einfachbindung oder für -(C=O)-,
wobei Asc steht für einen Ascorbinsäurerest der Formel Ila oder IIb 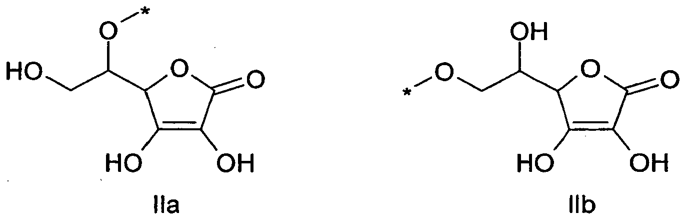
oder einen Dehydroascorbinsäurerest der Formeln IIc oder IId 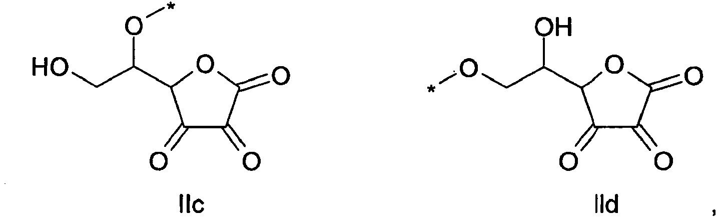
wobei zumindest ein R¹ bis R⁵ steht für NH₂, NHAlk, NAlk₂, NHArI oder NArl₂,
wobei die verbleibenden Substituenten R¹ bis R⁵ jeweils unabhängig voneinander stehen für H, OH, NH₂, Alk, OAIk, NHAlk, NAIk₂, NHArl oder NArl₂,
wobei Alk jeweils unabhängig voneinander steht für eine geradkettige oder verzweigte oder cyclische C₁- bis C₂₀-Alkylgruppe,
wobei Arl jeweils unabhängig voneinander steht für eine unsubstituierte, einfach- oder mehrfachsubstituierte C₆- bis C₂₀-Arylgruppe, die mit OH, Alk, OAlk oder NAlk₂ substituiert sein kann,
und/oder deren Salze, Tautomere, Stereoisomere, einschließlich deren Mischungen in allen Verhältnissen, als Oxidationsfarbstoffkomponente zum Färben einer Matrix.

2. Verwendung nach Anspruch 1,
**dadurch gekennzeichnet, dass**
Asc in der Formel I für einen Ascorbinsäurerest nach Formel Ilb steht.

3. Verwendung nach Anspruch 1 oder 2,
**dadurch gekennzeichnet, dass**
Y in der Formel I für -(C=O)- steht.

4. Verwendung nach einem oder mehreren der Ansprüche 1 bis 3,
**dadurch gekennzeichnet, dass**
X in der Formel I für -(CH₂)ₙ- mit 1≤n≤4 oder für eine Einfachbindung steht.

5. Verwendung nach einem oder mehreren der Ansprüche 1 bis 4,
**dadurch gekennzeichnet, dass**
X in der Formel I für -(CH₂)- oder für eine Einfachbindung steht.

6. Verwendung nach einem oder mehreren der Ansprüche 1 bis 5,
**dadurch gekennzeichnet, dass**
die Substituenten R¹ bis R⁵ in der Formel I jeweils unabhängig voneinander für H, NH₂, NHAlk, NAlk₂, NHArl oder NArl₂ stehen.

7. Verwendung nach einem oder mehreren der Ansprüche 1 bis 6,
**dadurch gekennzeichnet, dass**
die Substituenten R¹ bis R⁵ in der Formel I jeweils unabhängig voneinander für H, NH₂, NAlk₂ oder NArl₂ stehen.

8. Verwendung nach einem oder mehreren der Ansprüche 1 bis 7,
**dadurch gekennzeichnet, dass**
die Substituenten R² und R⁵ in der Formel I für H oder Alk stehen.

9. Verwendung nach einem oder mehreren der Ansprüche 1 bis 8,
**dadurch gekennzeichnet, dass**
der Substituenten R⁴ in der Formel I für H oder Alk steht.

10. Verfahren zum Färben einer Matrix, bei dem in einem Färbeschritt die Matrix mit einer Dispersion und/oder einer Lösung und/oder einer Emulsion behandelt wird, die zumindest eine Verbindung der Formel I, wie in einem oder mehreren der Ansprüche 1 bis 9 beschrieben, als Entwicklungskomponente aufweist, wobei während des Färbeschrittes zumindest eine Kupplungskomponente mit zumindest einer Verbindung der Formel I oder gegebenenfalls mindestens einer weiteren Entwicklungskomponente zu einem die Matrix färbenden Oxidationsfarbstoff oder dessen Vorstufe reagiert.

11. Verfahren nach Anspruch 10,
**dadurch gekennzeichnet, dass**
die Matrix in einem Vorbehandlungsschritt zur Beeinflussung des Färbeverhaltens mittels eines Vorbehandlungsmittels vorbehandelt wird, wobei der Vorbehandlungsschritt parallel zum Färbeschritt durchgeführt werden kann.

12. Verbindungen der Formel I 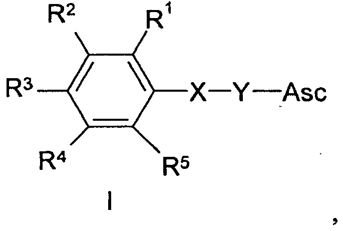
wobei X steht für eine Einfachbindung oder -(CH₂)ₙ-[O-(CH₂)ₘ]ₚ- mit 0≤n≤20, 0≤m≤20, 0p≤20 und 0≤n+m·p≤20,
wobei Y steht für eine Einfachbindung oder für -(C=O)-,
wobei Asc steht für einen Ascorbinsäurerest der Formel IIa oder IIb 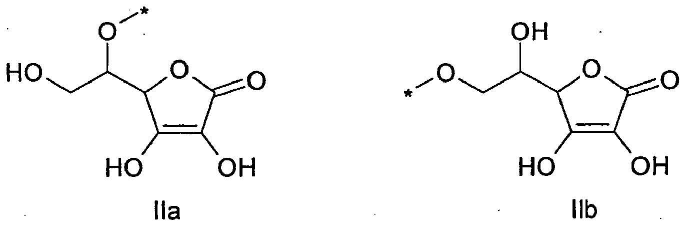
oder einen Dehydroascorbinsäurerest der Formeln IIc oder IId 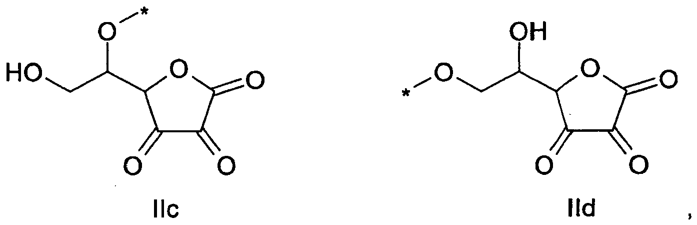
wobei zumindest ein R¹ bis R⁵ steht für NH₂, NHAlk, NAlk₂, NHArl oder NArl₂,
wobei die verbleibenden Substituenten R¹ bis R⁵ jeweils unabhängig voneinander stehen für H, OH, NH₂, Alk, OAlk, NHAlk, NAlk₂, NHArl oder NArl₂,
wobei Alk jeweils unabhängig voneinander steht für eine geradkettige oder verzweigte oder cyclische C₁- bis C₂₀-Alkylgruppe,
wobei Arl jeweils unabhängig voneinander steht für eine unsubstituierte, einfach- oder mehrfachsubstituierte C₆- bis C₂₀-Arylgruppe,
die mit OH, Alk, OAlk oder NAlk₂ substituiert sein kann,
und/oder deren Salze, Tautomere, Stereoisomere, einschließlich deren Mischungen in allen Verhältnissen, mit der Maßgabe, wenn X für eine Einfachbindung steht und Y für -(C=O)- steht, zumindest ein R¹ bis R⁵ für NHArl oder NArl₂ steht.

13. Zubereitung enthaltend zumindest einen für kosmetische, pharmazeutische, dermatologische Zubereitungen oder Haushaltsprodukte geeigneten Träger und zumindest eine Verbindung der Formel I nach Anspruch 12.

14. Zubereitung nach Anspruch 13,
**dadurch gekennzeichnet, dass**
die Zubereitung als Mehrkomponentensystem ausgebildet ist, wobei zumindest eine Verbindung der Formel I nach Anspruch 12, zumindest eine Kupplungskomponente, optional eine weitere Entwicklungskomponente, optional ein Vorbehandlungsmittel und zumindest ein Oxidationsmittel auf zumindest zwei Zubereitungskomponenten verteilt sind.

15. Verfahren zur Herstellung einer Zubereitung nach Anspruch 13, wobei zumindest eine Verbindung nach Formel I mit zumindest einem für kosmetische, pharmazeutische, dermatologische Zubereitungen oder Haushaltsprodukte geeigneten Träger und gegebenenfalls Hilfsstoffen und/oder Füllstoffen gemischt, insbesondere dispergiert und/oder emulgiert und/oder gelöst, wird.

## Claims

1. Use of compounds of the formula I 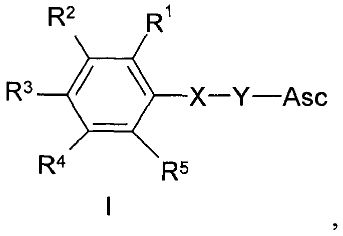
where X stands for a single bond or -(CH₂)ₙ-[O-(CH₂)ₘ]ₚ-, where 0≤n≤20, 0≤m≤20, 0≤p≤20 and 0≤n+m·p≤20,
where Y stands for a single bond or for -(C=O)-,
where Asc stands for an ascorbic acid radical of the formula Ila or Ilb 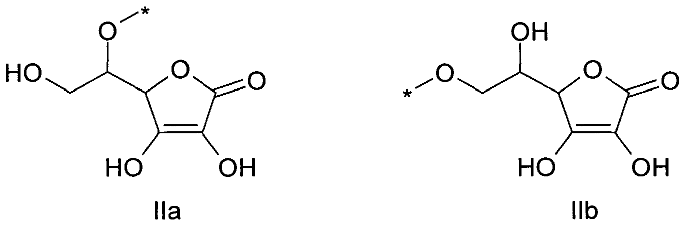
or a dehydroascorbic acid radical of the formula Ilc or Ild 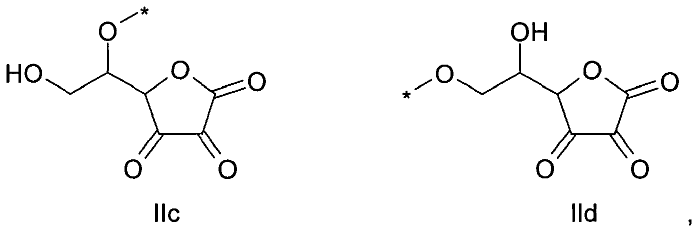
where at least one R¹ to R⁵ stands for NH₂, NHAlk, NAlk₂, NHArl or NArl₂,
where the remaining substituents R¹ to R⁵ each stand, independently of one another, for H, OH, NH₂, Alk, OAlk, NHAlk, NAlk₂, NHArl or NArl₂,
where Alk, in each case independently of one another, stands for a straight-chain or branched or cyclic C₁- to C₂₀-alkyl group,
where Arl, in each case independently of one another, stands for an unsubstituted, mono- or polysubstituted C₆- to C₂₀-aryl group, which may be substituted by OH, Alk, OAlk or NAlk₂,
and/or salts, tautomers, stereoisomers thereof, including mixtures thereof in all ratios, as oxidation dye component for dyeing a matrix.

2. Use according to Claim 1,
**characterised in that**
Asc in the formula I stands for an ascorbic acid radical of the formula IIb.

3. Use according to Claim 1 or 2,
**characterised in that**
Y in the formula I stands for -(C=O)-.

4. Use according to one or more of Claims 1 to 3,
**characterised in that**
X in the formula I stands for -(CH₂)ₙ-, where 1≤n≤4, or for a single bond.

5. Use according to one or more of Claims 1 to 4,
**characterised in that**
X in the formula I stands for -(CH₂)- or for a single bond.

6. Use according to one or more of Claims 1 to 5,
**characterised in that**
the substituents R¹ to R⁵ in the formula I each stand, independently of one another, for H, NH₂, NHAlk, NAlk₂, NHArl or NArl₂.

7. Use according to one or more of Claims 1 to 6,
**characterised in that**
the substituents R¹ to R⁵ in the formula I each stand, independently of one another, for for H, NH₂, NAlk₂ or NArl₂.

8. Use according to one or more of Claims 1 to 7,
**characterised in that**
the substituents R² and R⁵ in the formula I stand for H or Alk.

9. Use according to one or more of Claims 1 to 8,
**characterised in that**
the substituent R⁴ in the formula I stands for H or Alk.

10. Method for dyeing a matrix, in which, in a dyeing step, the matrix is treated with a dispersion and/or a solution and/or an emulsion which comprises at least one compound of the formula I, as described in one or more of Claims 1 to 9, as development component, where at least one coupling component reacts with at least one compound of the formula I or optionally at least one further development component during the dyeing step to give a matrix-dyeing oxidation dye or precursor thereof.

11. Method according to Claim 10,
**characterised in that**
the matrix is pre-treated by means of a pre-treatment agent in a pre-treatment step in order to influence the dyeing behaviour, where the pre-treatment step can be carried out in parallel with the dyeing step.

12. Compounds of the formula I 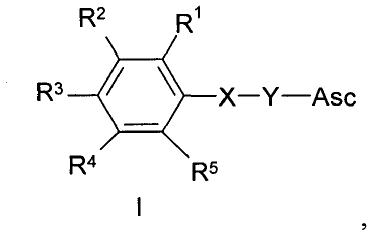
where X stands for a single bond or -(CH₂)ₙ-(O-(CH₂)ₘ]ₚ-, where 0≤n≤20, 0≤m≤20, 0≤p≤20 and 0≤n+m·p≤20,
where Y stands for a single bond or for -(C=O)-,
where Asc stands for an ascorbic acid radical of the formula Ila or Ilb 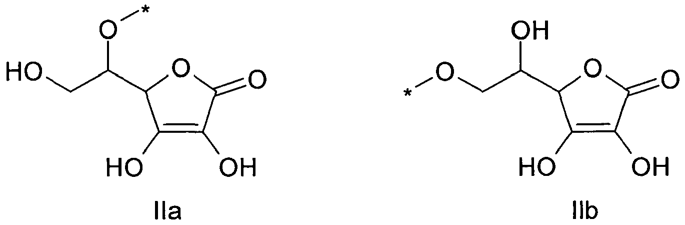
or a dehydroascorbic acid radical of the formula Ilc or Ild 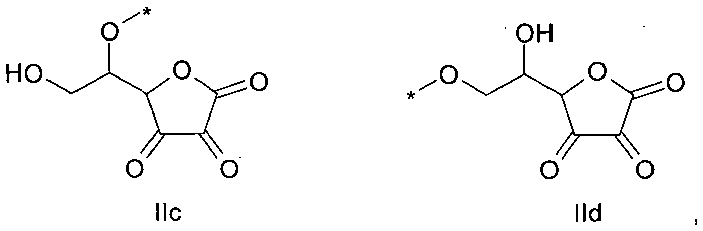
where at least one R¹ to R⁵ stands for NH₂, NHAlk, NAlk₂, NHArl or NArl₂,
where the remaining substituents R¹ to R⁵ each stand, independently of one another, for H, OH, NH₂, Alk, OAlk, NHAlk, NAlk₂, NHArl or NArl₂,
where Alk, in each case independently of one another, stands for a straight-chain or branched or cyclic C₁- to C₂₀-alkyl group,
where Arl, in each case independently of one another, stands for an unsubstituted, mono- or polysubstituted C₆- to C₂₀-aryl group, which may be substituted by OH, Alk, OAlk or NAlk₂,
and/or salts, tautomers, stereoisomers thereof, including mixtures thereof in all ratios, with the proviso that, if X stands for a single bond and Y stands for -(C=O)-, at least one R¹ to R⁵ stands for NHArl or NArl₂.

13. Preparation comprising at least one vehicle which is suitable for cosmetic, pharmaceutical, dermatological preparations or household products and at least one compound of the formula I according to Claim 12.

14. Preparation according to Claim 13,
**characterised in that**
the preparation has been formed as a multicomponent system, where at least one compound of the formula I according to Claim 12, at least one coupling component, optionally a further development component, optionally a pre-treatment agent and at least one oxidant are distributed over at least two preparation components.

15. Process for the preparation of a preparation according to Claim 13, where at least one compound of the formula I is mixed, in particular dispersed and/or emulsified and/or dissolved, with at least one vehicle which is suitable for cosmetic, pharmaceutical, dermatological preparations or household products and optionally assistants and/or fillers.

## Revendications

1. Utilisation de composés de formule I 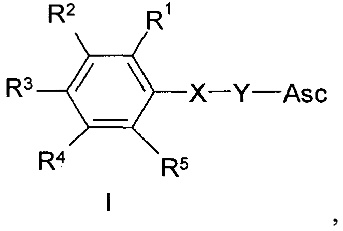
dans laquelle X représente une liaison simple ou -(CH₂)ₙ-[O-(CH₂)ₘ]ₚ-, où 0≤n≤20, 0≤m≤20, 0≤p≤20 et 0≤n+m·p≤20,
où Y représente une liaison simple ou-(C=O)-,
où Asc représente un radical d'acide ascorbique de formule Ila ou Ilb 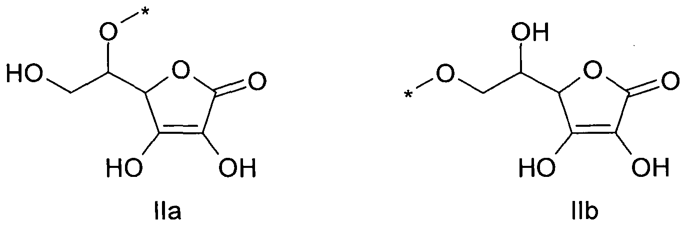
ou un radical d'acide déshydroascorbique de formule Ilc ou Ild 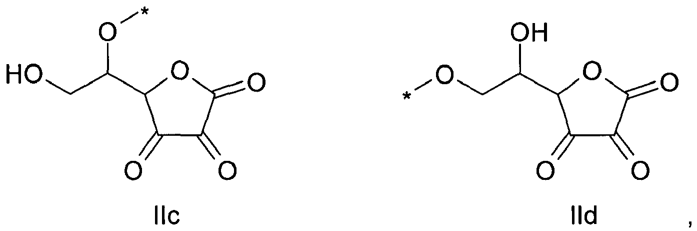
où au moins l'un parmi R¹ à R⁵ représente NH₂, NHAlk, NAlk₂, NHArl ou NArl₂,
où les substituants restants R¹ à R⁵ représentent chacun, indépendamment les uns des autres, H, OH, NH₂, Alk, OAlk, NHAlk, NAlk₂, NHArl ou NArl₂,
où Alk, dans chaque cas indépendamment les uns des autres, représente un groupement C₁- à C₂₀-alkyle à chaîne linéaire ou ramifiée ou cyclique,
où Arl, dans chaque cas indépendamment les uns des autres, représente un groupement C₆- à C₂₀-aryle non substitué, mono- ou polysubstitué, qui peut être substitué par OH, Alk, OAlk ou NAlk₂, et/ou des sels, tautomères, stéréoisomères de ceux-ci, y compris des mélanges de ceux-ci en toutes proportions, comme composant de colorant d'oxydation pour la coloration d'une matrice.

2. Utilisation selon la revendication 1,
**caractérisée en ce que**
Asc dans la formule I représente un radical d'acide ascorbique de formule Ilb.

3. Utilisation selon la revendication 1 ou 2,
**caractérisée en ce que**
Y dans la formule I représente -(C=O)-.

4. Utilisation selon l'une ou plusieurs parmi les revendications 1 à 3,
**caractérisée en ce que**
X dans la formule I représente -(CH₂)ₙ-, où 1≤n≤4, ou une liaison simple.

5. Utilisation selon l'une ou plusieurs parmi les revendications 1 à 4,
**caractérisée en ce que**
X dans la formule I représente -(CH₂)- ou une liaison simple.

6. Utilisation selon l'une ou plusieurs parmi les revendications 1 à 5,
**caractérisée en ce que**
les substituants R¹ à R⁵ dans la formule I représentent chacun, indépendamment les uns des autres, H, NH₂, NHAlk, NAlk₂, NHArl ou NArl₂.

7. Utilisation selon l'une ou plusieurs parmi les revendications 1 à 6,
**caractérisée en ce que**
les substituants R¹ à R⁵ dans la formule I représentent chacun, indépendamment les uns des autres, H, NH₂, NAlk₂ ou NArl₂.

8. Utilisation selon l'une ou plusieurs parmi les revendications 1 à 7,
**caractérisée en ce que**
les substituants R² et R⁵ dans la formule 1 représentent H ou Alk.

9. Utilisation selon l'une ou plusieurs parmi les revendications 1 à 8,
**caractérisée en ce que**
le substituant R⁴ dans la formule I représente H ou Alk.

10. Méthode de coloration d'une matrice, **caractérisée en ce que**, dans une étape de coloration, la matrice est traitée par une dispersion et/ou une solution et/ou une émulsion comprenant au moins un composé de formule I, tel que décrit selon l'une ou plusieurs parmi les revendications 1 à 9, comme composant de développement, où au moins un composant de couplage réagit avec au moins un composé de formule I ou éventuellement au moins un autre composant de développement pendant l'étape de coloration pour donner un colorant d'oxydation de coloration de matrice ou un précurseur de celui-ci.

11. Méthode selon la revendication 10,
**caractérisée en ce que**
la matrice est prétraitée au moyen d'un agent de prétraitement dans une étape de prétraitement afin d'influencer le comportement de coloration, où l'étape de prétraitement peut être effectuée en parallèle avec l'étape de coloration.

12. Composés de formule I 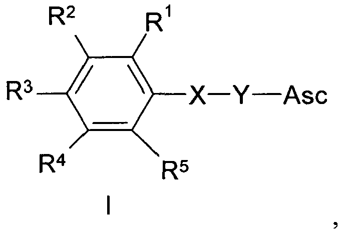
dans laquelle X représente une liaison simple ou -(CH₂)ₙ-[O-(CH₂)ₘ]ₚ-, où 0≤n≤20, 0≤m≤20, 0≤p≤20 et 0≤n+m·p≤20,
où Y représente une liaison simple ou-(C=O)-,
où Asc représente un radical d'acide ascorbique de formule IIa ou IIb 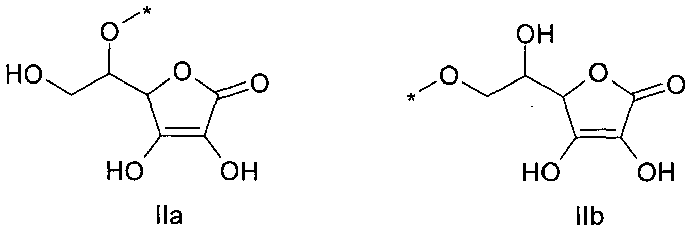
ou un radical d'acide déshydroascorbique de formule Ilc ou Ild 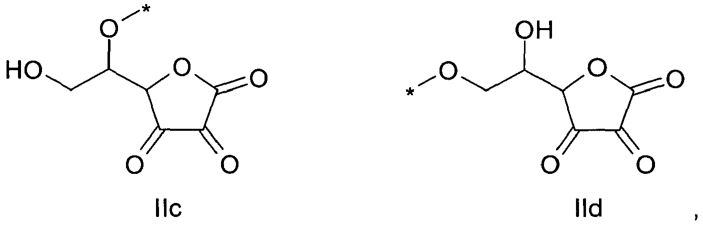
où au moins l'un parmi R¹ à R⁵ représente NH₂, NHAlk, NAlk₂, NHArI ou NArl₂,
où les substituants restants R¹ à R⁵ représentent chacun, indépendamment les uns des autres, H, OH, NH₂, Alk, OAlk, NHAlk, NAlk₂, NHArl ou NArl₂,
où Alk, dans chaque cas indépendamment les uns des autres, représente un groupement C₁- à C₂₀-alkyle à chaîne linéaire ou ramifiée ou cyclique,
où Arl, dans chaque cas indépendamment les uns des autres, représente un groupement C₆- à C₂₀-aryle non substitué, mono- ou polysubstitué, qui peut être substitué par OH, Alk, OAlk ou NAlk₂,
et/ou des sels, tautomères, stéréoisomères de ceux-ci, y compris des mélanges de ceux-ci en toutes proportions, à condition que, si X représente une liaison simple et Y représente -(C=O)-, au moins l'un parmi R¹ à R⁵ représente NHArl ou NArl₂.

13. Préparation comprenant au moins un véhicule qui est convenable pour des préparations cosmétiques, pharmaceutiques, dermatologiques ou des produits ménagers et au moins un composé de formule I selon la revendication 12.

14. Préparation selon la revendication 13,
**caractérisée en ce que**
la préparation a été formée comme système multi-composants, où au moins un composé de formule I selon la revendication 12, au moins un composant de couplage, éventuellement un autre composant de développement, éventuellement un agent de prétraitement et au moins un oxydant sont distribués sur au moins deux composants de préparation.

15. Procédé de préparation d'une préparation selon la revendication 13, **caractérisé en ce qu'**au moins un composé de formule I est mélangé, en particulier dispersé et/ou émulsifié et/ou solubilisé, avec au moins un véhicule qui est convenable pour des préparations cosmétiques, pharmaceutiques, dermatologiques ou des produits ménagers et éventuellement des auxiliaires et/ou des charges.
